# EUROPÄISCHE PATENTSCHRIFT

(11) **EP 1 017 660 B1**
(45) Veröffentlichungstag und Bekanntmachung des Hinweises auf die Patenterteilung: **18.12.2002**
(21) Anmeldenummer: 98948928.1
(22) Anmeldetag: 08.09.1998
(51) Int. Cl.: C07C 45/50

(54) **VERFAHREN ZUR HERSTELLUNG VON ALDEHYDEN DURCH HYDROFORMYLIERUNG IN GEGENWART VON KRONENETHER**
METHOD FOR PRODUCING ALDEHYDES BY HYDROFORMYLATION IN THE PRESENCE OF CROWN ETHER
PROCEDE DE PRODUCTION D'ALDEHYDES PAR HYDROFORMYLATION EN PRESENCE D'ETHER COURONNE

(30) Priorität: 25.09.1997 DE 19742305
(43) Veröffentlichungstag der Anmeldung: 12.07.2000
(73) Patentinhaber: Celanese Chemicals Europe GmbH, 60439 Frankfurt am Main (DE)
(72) Erfinder: BOGDANOVIC, Sandra, D-60322 Frankfurt (DE); KÜHLEIN, Klaus, D-65779 Kelkheim (DE)
(86) Internationale Anmeldenummer: EP9805683
(87) Internationale Veröffentlichungsnummer: WO99015488

(56) Entgegenhaltungen:
- EP-A- 0 302 375
- EP-A- 0 491 239
- EP-A- 0 571 819
- US-A- 4 399 312
- US-A- 4 731 486

## Beschreibung

Die vorliegende Erfindung betrifft ein Verfahren zur Hydroformylierung olefinisch ungesättigter Verbindungen mit 3 bis 12 Kohlenstoffatomen mit Wasserstoff und Kohlenmonoxid bei erhöhtem Druck.

Bei der Hydroformylierung von Olefinen mit Kohlenmonoxid und Wasserstoff werden Aldehyde und Alkohole hergestellt, wobei die Aldehyde und Alkohole ein Kohlenstoffatom mehr als das Ausgangsolefin enthalten. Der verwendete Katalysator wird hierbei üblicherweise in homogener Phase mit dem Olefin eingesetzt. Die Reaktion wird vorzugsweise durch Hydridometallcarbonyle der Metalle der VIII. Gruppe des Periodensystems katalysiert. Neben Kobalt, das als Katalysatormetall in großem Umfang technische Anwendung findet, hat Rhodium vorzugsweise für die Hydroformylierung niederer Olefine zunehmend Bedeutung erlangt. Im Gegensatz zu Kobalt gestattet Rhodium, die Reaktion bei niedrigem Druck durchzuführen, darüber hinaus werden bei Einsatz endständiger Olefine bevorzugt geradkettige n-Aldehyde und nur in untergeordnetem Maße iso-Aldehyde gebildet. Darüber hinaus ist auch die Hydrierung der olefinischen Verbindungen zu gesättigten Kohlenwasserstoffen in Gegenwart von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. Ihre Anwendung ermöglicht, den Reaktionsdruck auf Werte unter 30 MPa zu senken.

So betriftt die DE-PS 27 43 630 die Herstellung von Alkanpolyolen (Glykolen) aus Synthesegas unter Verwendung eines mindestens 4 Sauerstoffatome enthaltenden Kronenethers als Lösemittel. Dieser Kronenether dient zur Abtrennung von Ionen aus der homogenen, flüssigen Phase ohne gleichzeitige Komplexbildung des rhodiumhaltigen Katalysators. Die Reaktionstemperaturen liegen bei allen Beispielen über 220°C.
In der US-A-4 320 064 wird die Reaktion von Kohlenmonoxid mit Wasserstoff zu Polyolen unter Verwendung von Rhodiumcarbonyl-Clustem in homogener flüssiger Phase beschrieben. Als Katalysator wird ein durch 18-Krone-6 komplexiertes Cäsiumsalz von [Rh₂₂(CO)₃₅Hₓ]ⁿ⁻ verwendet. Auch hier dient der Kronenether zur Kompfexierung des kationischen Bestandteils der Rhodiumcarbonyl-Cluster. Die in den Beispielen angegebenen Temperaturen liegen zwischen 250 und 270°C.

Probleme wirft bei diesen Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung kurzkettiger Olefine mit 3 bis 5 Kohlenstoffatomen beschritten werden.
Bei der Hydroformylierung von Olefinen mit mehr als 6 Kohlenstoffatomen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.
Das Problem der thermischen Zersetzung wird vermieden, wenn man eine Zweiphasenkatalyse verwendet. Hierbei liegen zwei flüssige, miteinander nicht mischbare Phasen vor, von denen die eine organische Phase das Olefin enthält und die andere, zumeist polare Phase, den Katalysator enthält. Voraussetzung für die Anwendung dieses Verfahrens ist die Löslichkeit des Katalysators in der polaren Phase. Im industriellen Maßstab wird als polare Phase eine wäßrige Phase und als Katalysator eine Rhodiumkomplexverbindung verwendet. Die Löslichkeit des Katalysators in der wäßrigen Phase wird hierbei durch Verwendung sulfonierter Triarylphosphine als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Ein solches Verfahren ist beispielsweise in der DE-PS 26 27 354 beschrieben. Ein besonderes Merkmal dieser Arbeitsweise ist, daß aus endständigen Olefinen mit hoher Selektivität n-Aldehyde und nur in untergeordnetem Maße iso-Aldehyde (d.h. in α-Position zur Aldehydgruppe verzweigte Aldehyde) gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich auch bei der Hydroformylierung niederer Olefine, insbesondere Propen und Buten, bewährt. Setzt man höhere Olefine wie Penten oder Hexen ein, geht jedoch die Umsetzungsgeschwindigkeit bereits merklich zurück. Die Wirtschaftlichkeit der Umsetzung in technischem Maßstab ist bei Einsatz von Olefinen mit mehr als vier Kohlenstoffatomen häufig nicht mehr in gewünschtem Umfang gegeben.

Um bei der Hydroformylierung von Olefinen mit mehr als 5 Kohlenstoffatomen mittels wasserlöslicher Katalysatoren den Umsatz und/oder die Selektivität der Reaktion zu n-Aldehyden zu steigern, wurden auch spezielle amphiphile Reagenzien oder Lösungsvermittler eingesetzt.
Der Zusatz dieser Stoffe führt dazu, daß der Stofftransport zwischen den einzelnen Phasen und damit die Mischbarkeit von wäßriger Katalysatorphase und organischer Phase begünstigt werden.

So betrifft die DE 34 12 335 die Hydroformylierung von Olefinen unter Verwendung quaternärer Ammoniumsalze. Wie aus Tabelle 4 hervorgeht, führt die Hydroformylierung von Hexen mittels Rhodium und Trinatrium-tris(m-sulfophenyl)phosphin ohne Zusatz eines Lösungsvermittlers zu einem Umsatz von 36 % (Beispiel 10), während ein Zusatz von Polyolen als Lösungsvermittler (Bsp. 11: 5 % Polyglykol 200; Bsp.14: 2,5 % Triethylenglykol) nur einen Umsatz von 43,5 % bzw. 43 % bewirkt. Ein sehr hoher Umsatz, nämlich 86 %, wird hingegen durch Zusatz von 2,5 % Trimethylhexadecylammoniumbromid als Lösungsvermittler erzielt. Diese Druckschrift zeigt, daß erst der Zusatz quaternärer Ammoniumsalze eine beachtliche Steigerung des Umsatzes zur Folge hat. Dementgegen hat weder der Zusatz von Tri- oder Polyglykolen, noch eine Erhöhung der Menge dieser Stoffe um das Doppelte (von 2,5 auf 5 %), eine signifikante Steigerung des Umsatzes zur Folge.

In der DE 31 35 127 A1 wird die Hydroformylierung von Olefinen unter Verwendung amphiphiler Reagenzien in Gegenwart eines durch einen Phosphinliganden komplexierten Rhodiumkatalysators beschrieben. Die Tabelle 7 zeigt, daß die Hydroformylierung von 1-Dodecen mittels Rhodium und monosulfoniertem Triphenylphosphin (3-Ph₂PC₆H₄SO₃Na) ohne Zusatz eines amphiphilen Reagenz zu einem Umsatz von 56 % (Beispiel 77) führt, während der Zusatz von 18-Krone-6 zu einer Minderung des Umsatzes auf 40 % (Beispiel 57) führt. Auch hier werden hohe Ausbeuten erst durch Verwendung quaternärer Ammoniumsalze erzielt (Bsp. 68: 85 % mit CTAB).

Ein wesentlicher Nachteil bei der Verwendung quaternärer Ammoniumsalze als amphiphile Reagenzien liegt allerdings in ihrer schlechten biologischen Abbaubarkeit. So führt beispielsweise die Anwesenheit quaternärer Ammoniumsalze im Abwasser zu erheblichen Schwierigkeiten bei der Abwasseraufbereitung.

Ein weiterer Nachteil bei der Verwendung quarternärer Ammoniumsalze als Lösungsvermittler liegt darin, daß die mit diesen Verbindungen erreichte Steigerung der Vermischbarkeit von wäßriger Katalysatorphase und organischer Phase mit einer erhöhten Löslichkeit von organischer Phase in wäßriger Phase und von wäßriger Phase in organischer Phase einhergeht. Auf diese Weise kann in zunehmendem Maße sowohl amphiphiles Reagenz und Lösungsvermittler als auch Rhodium und wasserlösliches Phosphin in die organische Phase gelangen und nach Phasentrennung mit der organischen Phase ausgetragen werden.
Der Austrag dieser Substanzen über die organische Phase ist natürlich unerwünscht, da im gleichen Maße neue Substanzen wieder zur wäßrigen Phase hinzugegeben werden müssen, was insbesondere in Hinblick auf Rhodium mit einem erheblichen finanziellen Mehraufwand verbunden ist.

Desweiteren findet mit höherem Zusatz amphiphiler Reagenzien oder Lösungsvermittler - d.h. mit steigender Vermischbarkeit von wäßriger Katalysatorphase und organischer Phase - die für die Phasentrennung erforderliche Entmischung infolge der Bildung von Emulsionen oder Lösungen nicht mehr oder nicht in genügendem Umfang statt. Dies ist insbesondere bei solchen amphiphilen Reagenzien der Fall, die auch als Tenside oder Schaumbildner eingesetzt werden können.
Dies ist insofern von Nachteil, als eine gute Entmischbarkeit eine unabdingbare Voraussetzung für die erforderliche, die Hydroformylierung abschließende Trennung von organischer und wäßriger Phase ist.

Die Aufgabe der Erfindung besteht in der Bereitstellung eines Verfahrens zur Herstellung von Aldehyden durch Hydroformylierung von Olefinen mit 3 bis 12 Kohlenstoffatomen, das höhere Umsätze im Vergleich zum Stand der Technik ermöglicht und die Nachteile des Standes der Technik vermeidet.
Diese Aufgabe wird gelöst durch ein Verfahren zur Herstellung von Aldehyden durch Umsetzung eines Olefins mit 3 bis 12 Kohlenstoffatomen mit Wasserstoff und Kohlenmonoxid in Gegenwart eines Rhodiumkatalysators, dadurch gekennzeichnet, daß
(a) der Rhodiumkatalysator in einer wäßrigen Phase vorliegt, wobei die wäßrige Phase
   i) Rhodium in elementarer oder gebundener Form;
   ii) ein trisulfoniertes Triarylphosphin; und
   iii) einen Kronenether enthält;
   und
b) das Olefin unter Reaktionsbedingungen in einer flüssigen organischen Phase vorliegt, die mit der wäßrigen Phase nicht mischbar ist.

Das erfindungsgemäße Verfahren zeichnet sich insbesondere dadurch aus, daß im Vergleich zum Stand der Technik höhere Umsätze bei einer hohen Selektivität erreicht werden und daß kein signifikanter Anstieg von Rhodium oder Liganden in der organischen Phase und somit kein erhöhter Austrag des Katalysators über die organische Phase beobachtet wird.

Darüber hinaus ist die Entmischbarkeit von organischer Phase und wäßriger Katalysatorphase so hoch, daß eine schnelle Trennung von organischer Phase und wäßriger Phase gewährleistet ist. Bei Anwendung dieses Verfahrens werden weder schwertrennbare Emulsionen noch nicht trennbare, homogene Lösungen gebildet.

Die erfindungsgemäß zu verwendenden Kronenether sind z.B. in "Ullmann's Encyclopedia of Industrial Chemistry" Weinheim (1987), 5^{th} Ed., Band A8, S.91-97 beschrieben.

Bei dem trisulfonierten Triarylphosphin handelt es sich entsprechend einer bevorzugten Ausführungsform um ein trisulfoniertes Triarylphosphin der Formel (I) 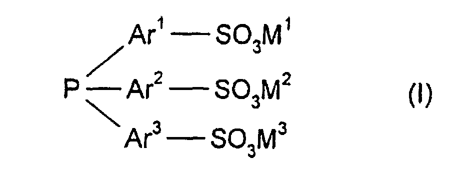 in der
Ar¹, Ar² und Ar³ unabhängig voneinander jeweils einen Phenyl-, Naphtyl-, Biphenyl-, Phenylnaphtyl- oder Binaphtylrest darstellen;
M¹, M² und M³ unabhängig voneinander jeweils ein Alkalimetallion oder ein Ammoniumion darstellen.

Es ist aber auch möglich, daß M¹, M² und M³ andere, höherwertige Kationen darstellen wie beispielsweise Erdalkali- oder Zinkkationen, wobei der Ladungsausgleich maßgeblich die Anzahl dieser Kationen bestimmt.

Dieses trisulfonierte Triarylphosphin der Formel (I) ist besonders bevorzugt Trinatrium-tris(m-sulfophenyl)phosphin der Formel

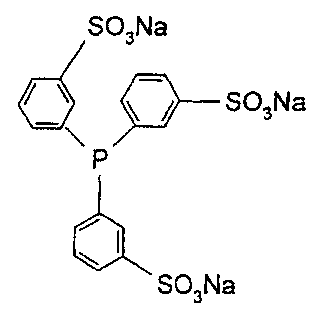

Dieses Trinatriumsalz enthält aufgrund seiner Herstellung durch Sulfonierung von Triphenylphosphin Anteile an mono- und disulfonierten Verbindungen und geringe Anteile der entsprechenden Phosphinoxide.

Das trisulfonierte Triarylphosphin kann auch ein sulfoniertes Triarylphosphin mit zwei Phosphoratomen sein, das beispielsweise einen Rest -(CH₂)ₓ-Ar-Ar-(CH₂)ₓ- enthält, worin
- x für eine ganze Zahl von 1 bis 4, insbesondere 1 bis 2, vorzugsweise 1 steht;
- Ar-Ar Biphenyl oder Binaphthyl bedeutet;
- die -(CH₂)ₓ-Gruppe mit der einen Bindung jeweils in ortho-Stellung zu der die beiden Arylreste verbindenden Aryl-Arylbindung Ar-Ar steht, und mit der anderen Bindung jeweils mit einem Phosphoratom, das jeweils zwei weitere, gleiche oder verschiedene Arylreste, insbesondere Phenylreste besitzt, verbunden ist.

Beispiele für derartige, zwei Phosphoratome enthaltende, trisulfonierte Triarylphosphine sind Verbindungen der Formel (II) 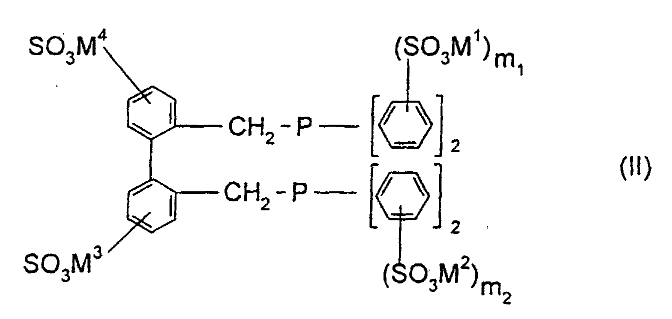 in der
- m¹ und m² den Wert 0 oder 1 haben können, wobei die Summe von m¹ und m² mindestens 1 beträgt; und
- M¹, M², M³ und M⁴ unabhängig voneinander jeweils ein Alkalimetallion oder ein Ammoniumion darstellen.

Es ist aber auch möglich, daß M¹, M², M³ und M⁴ andere, höherwertige Kationen darstellen wie beispielsweise Erdalkali- oder Zinkkationen, wobei der Ladungsausgleich maßgeblich die Anzahl dieser Kationen bestimmt.

Ebensogut kann das organische, zwei Phosphoratome enthaltende trisulfonierte Triarylphosphin eine Verbindung der Formel (III) sein, in der 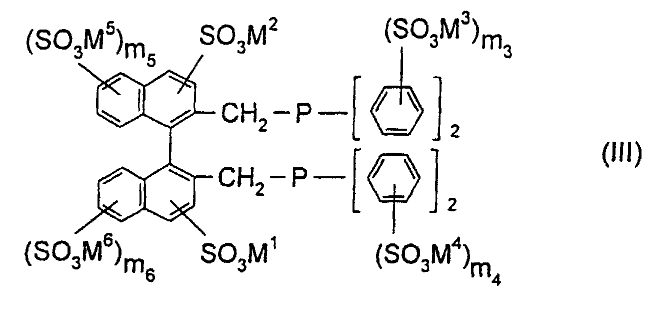
- m₃, m₄, m₅ und m₆ den Wert 0 oder 1 haben können, wobei die Summe von m₃, m₄, m₅ und m₆ mindestens 2 beträgt; und
- M¹, M², M³, M⁴, M⁵ und M⁶ unabhängig voneinander jeweils ein Alkalimetallion oder ein Ammoniumion darstellen.

Auch hier ist es möglich, daß M¹, M², M³, M⁴, M⁵ und M⁶ andere, höherwertige Kationen darstellen wie beispielsweise Erdalkali- oder Zinkkationen, wobei der Ladungsausgleich maßgeblich die Anzahl dieser Kationen bestimmt.

Solche zwei Phosphoratome enthaltende Triarylphosphine der Formeln (II) und (III) weisen insbesondere vier bis acht -SO₃M Reste, auf. Die -SO₃M Reste sitzen üblicherweise an den Arylresten des Restes -(CH₂)ₓ-Ar-Ar-(CH₂)ₓ- und an den zwei weiteren Arylresten, die mit dem Phosphor verbunden sind.

Alternativ können anstelle sulfonierter Triarylphosphine andere Triarylphosphine als Liganden eingesetzt werden, bei denen die SO₃M-Gruppe durch andere Gruppen ersetzt wird, die eine Wasserlöslichkeit des Triarylphosphins bewirken, wie z.B. PO₃M₂-Gruppen.

Gemäß einer besonders bevorzugten Ausführungsform ist der Kronenether ausgewählt aus der Gruppe von 12-Krone-4, 15-Krone-5, 18-Krone-6 und Hydroxymethyl-18-Krone-6.

Das Olefin kann aus aliphatischen, cycloaliphatischen und araliphatischen Olefinen, insbesondere aus aliphatischen und cycloaliphatischen α-Olefinen, ausgewählt sein.

Die olefinische Verbindung kann eine oder mehrere Kohlenstoff-Kohlenstoff-Doppelbindung enthalten. Die Kohlenstoff-Kohlenstoff-Doppelbindung kann endständig oder innenständig angeordnet sein. Bevorzugt sind olefinische Verbindungen mit endständiger Kohlenstoff-Kohlenstoff-Doppelbindung.
Beispiele für α-olefinische Verbindungen (mit endständiger Kohlenstoff-Kohlenstoff-Doppelbindung) sind 1-Alkene, Alkylalkenoate, Alkylenalkanoate, Alkenylalkylether und Alkenole.
Ohne Anspruch auf Vollständigkeit seien als α-olefinische Verbindungen Propen, 1-Buten, 1-Penten, 1-Hexen, 1-Hepten, 1-Octen, 1-Nonen, 1-Decen, 1-Undecen und 1-Dodecen genannt.
Besonders bevorzugt sind Olefine, die höchstens 10, vorzugsweise höchstens 8 Kohlenstoffatome aufweisen.
Im Hinblick auf einen industriellen Einsatz dieses Verfahrens ist das Olefin insbesondere ausgewählt aus der Gruppe von Propen, 1-Buten, 1-Penten und 1-Hexen.

Im Hinblick auf das erfindungsgemäße Verfahren hat es sich als günstig erwiesen, daß die wäßrige Phase 20 bis 500 ppm, vorzugsweise 30 bis 150 ppm, insbesondere 40 bis 100 ppm, Rhodium enthält.
Das Verhältnis von Rhodium zum Liganden kann hierbei zwischen 1:10 und 1:1000, vorzugsweise zwischen 1:50 und 1:200, liegen.

Die den Katalysator enthaltende, wäßrige Phase läßt sich auf vergleichsweise einfache Art herstellen, indem man ein wasserlösliches Rhodiumsalz, die trisulfonierten Triarylphosphine und die Verbindung der Formel (1) in Wasser löst. Geeignete Rhodiumsalze sind, ohne Anspruch auf Vollständigkeit zu erheben: Rhodium(III)sulfat, Rhodium(III)nitrat, Rhodium(III)carboxylate wie Rhodium(III)acetat, Rhodiumpropionat, Rhodiumbutyrat und Rhodium-2-ethylhexanoat.
Man kann die wäßrige Phase unmittelbar in die Hydroformylierung einsetzen oder sie zuvor einer Präformierung des Katalysators unter Reaktionsbedingungen unterwerfen, um sie anschließend in präformierter Form zu verwenden.

Die wäßrige Phase kann vorzugsweise 2 x 10⁻⁶ bis 5 x 10⁻² Mol Rhodium pro Mol olefinischer Verbindung enthalten.

Der Druck während der Reaktion liegt im allgemeinen zwischen 20 und 150 bar, vorzugsweise zwischen 30 und 80 bar. Das Verhältnis von Kohlenmonoxid und Wasserstoff kann in weiten Grenzen variieren. Günstig ist ein Verhältnis von Kohlenmonoxid zu Wasserstoff von 10:1 bis 1:30, insbesondere von 5:1 bis 1:8, bevorzugt von 1:2 bis 2:1. Besonders bevorzugt ist ein Zupressen von Synthesegas im Verhältnis Kohlenmonoxid zu Wasserstoff von 1:1.
Die Temperatur liegt während der Reaktion üblicherweise zwischen zwischen 20 und 170°C, vorzugsweise zwischen 100 und 140°C.

Als Reaktionsgefäße werden Druckreaktoren mit magnetischer oder mechanischer Rühr- oder Mischeinrichtung verwendet. Während der Umsetzung ist eine gute Durchmischung der vorhandenen Phasen, d.h. von polarer Phase, Kohlenmonoxid/Wasserstoff und ggf. organischer Phase sicherzustellen. Dies kann insbesondere durch intensives Rühren und/oder Umpumpen von organischer und wäßriger Phase bewirkt werden.
Eine kontinuierliche Führung des Versuches ist ebenfalls möglich.

Nach dem Ende der Reaktion wird der Druckreaktor gekühlt, durch Druckentspannung von Kohlenmonoxid und Wasserstoff befreit und das Reaktionsgemisch entnommen. Bei Abschalten der Durchmischungseinrichtung trennen sich die Phasen von allein innerhalb von Sekunden. Die organische Phase kann destillativ aufgearbeitet werden und dann bei Bedarf gaschromatographisch untersucht werden.

Die folgenden Beispiele dienen der Erläuterung der Erfindung.

### Beispiel 1 (Vergleichsbeispiel):

### a): Herstellung der Katalysatorphase und Präformierung

60 mg (0,23 mmol) Rhodium(III)acetat werden in 39 ml einer 0,6 M wäßrigen Lösung des Trinatriumsalzes von TPPTS (Tris-(metasulfonato)triphenylphosphin) entsprechend einem Molverhältnis Rhodium zu Ligand von 1:100 und 21 ml entgastem destillierten Wasser gelöst und in einen 200 ml Stahlautoklav unter Stickstoffstrom gegeben. Die so hergestellte Katalysatorlösung wird bei 25 bar Synthesegasdruck (CO/H₂ = 1/1) während 3 Stunden auf 125°C erwärmt, wobei der aktive Katalysatorkomplex entsteht und die Lösung sich intensiv gelb färbt.

### b): Hydroformylierung

Zu der präformierten Katalysatorlösung aus 1.a) werden unter einem Reaktionsdruck von 30 bar und bei 125 °C werden 26,3 ml 1-Penten (240 mmol) über einen vorgeschalteten 200 ml Stahlautoklav mit leichtem Überdruck zugegeben. Das Verhältnis von Olefin zu Rhodium beträgt 1030 :1. Die Hydroformylierungsreaktion wird durch Einschalten des Magnetrührers in Gang gesetzt. Während einer Reaktionszeit von 3 Stunden wird die Temperatur auf 125 °C gehalten und der Reaktionsdruck in einem Druckband von +/- 3 bar durch manuelles Zudosieren von Synthesegas konstant gehalten. Nach Ablauf von 3 Stunden werden Rührer und Heizung abgeschaltet, der Autoklav auf 40 bis 50 °C abgekühlt und die obere Produktphase von der Katalysatorphase mittels Phasentrennung in einem Scheidetrichter getrennt. Produktphase und Katalysatorphase werden gewogen. Die Zusammensetzung der Produktphase wird mittels Gaschromatographie und ¹H-NMR-Spektroskopie ermittelt und aus der Zusammensetzung die Ausbeute an Hydroformylierungsprodukten und das Verhältnis von n- zu iso-Heptanal bestimmt.
Die Ausbeute an Hydroformylierungsprodukten beträgt 49,4 %, das n/iso-Verhältnis beträgt 96:4.

### Beispiel 2:

### a): Herstellung der Katalysatorphase und Präformierung

30 mg (0,166 mmol) Rhodium(III)acetat werden in 19,5 ml einer 0,6 M wäßrigen Lösung des Trinatriumsalzes von TPPTS (Tris-(metasulfonato)triphenylphosphin) gelöst und mit 2,88 g des Kronenethers 18-Krone-6 versetzt. Diese Katalysatorphase wird unter Stickstoffstrom in einen 200 ml Stahtautoklav gegeben und bei 25 bar Synthesegasdruck (CO/H₂ = 1/1) während 3 Stunden auf 125 °C erwärmt.

### b): Hydroformylierung

Zu der präformierten Katalysatorlösung aus 2.a) werden 13,2 ml 1-Penten (120 mmol) zugegeben. Die Hydroformylierung wird analog Beispiel 1.a) bei 125°C und bei 50 bar Synthesegas durchgeführt. Die Zusammensetzung der Produktphase wird mittels Gaschromatographie und ¹H-NMR-Spektroskopie ermittelt und aus der Zusammensetzung der Umsatzgrad, die Selektivität ermittelt.
Die Ausbeute an Hydroformylierungsprodukt beträgt 86,7 %, das n/iso-Verhältnis beträgt 94:6.

### Beispiele 3 bis 28:

Die Vergleichsbeispiele 14 und 20 werden analog zu Beispiel 1 bei 125°C und 50 bar Synthesegas durchgeführt. Die erfindungsgemäßen Beispiele 3 bis 13, 15 bis 19 und 21 bis 24 werden analog zu Beispiel 2 durchgeführt. In allen erfindungsgemäßen Beispielen wird eine 0,6 M wäßrige Lösung des Trinatriumsalzes von TPPTS (Tris-(metasulfonato)triphenylphosphin) entsprechend einem Molverhältnis von Rhodium zu Ligand von 1 : 100 verwendet. Die Angaben bezüglich der Mengen an Rhodium(III)acetat und TPPTS-Lösung, das Verhältnis von Olefin zu Rhodium sowie die Edukte und Reaktionsergebnisse sind der Tabelle 1 zu entnehmen.

In der Tabelle 1 werden folgende Bezeichnungen verwendet:
· "Olefin" bezeichnet das verwendete Olefin;
· "Rh-Kat." steht für die Menge an Rhodiumkatalysator in mmol;
· "Ol./Rh-Verh." steht für das molare Verhältnis von Olefin zu Rhodium;
· "TPPTS" steht für die Menge an eingesetzter 0,6 M wäßriger Lösung von Tris(metasulfonato)triphenylphosphin in ml;
· "Krone" steht für den verwendeten Kronenether und dessen Menge, hierbei steht 12-K-4 für 12-Krone-4, 15-K-5 für 15-Krone-5, 18-K-6 für 18-Krone-6 und 18-K-6-OH für Hydroxymethyl-18-Krone-6 ((CH₂-CH₂-O)₅-CH₂-CH(CH₂OH)O);
· "Gew.-% Krone" steht für den Anteil an eingesetztem Kronenether, bezogen auf die Katalysatorphase;
· "H₂O" steht für Menge an der wäßrigen Phase zugesetztem Wasser in ml;
· "t" steht für die Reaktionszeit in Minuten;
· "n : iso" steht für Verhältnis von n-Aldehyden zu iso-Aldehyden; "n.b." (nicht bestimmbar) bedeutet hierbei, daß sich das Verhältnis aufgrund des geringen Umsatzes nicht bestimmen läßt.

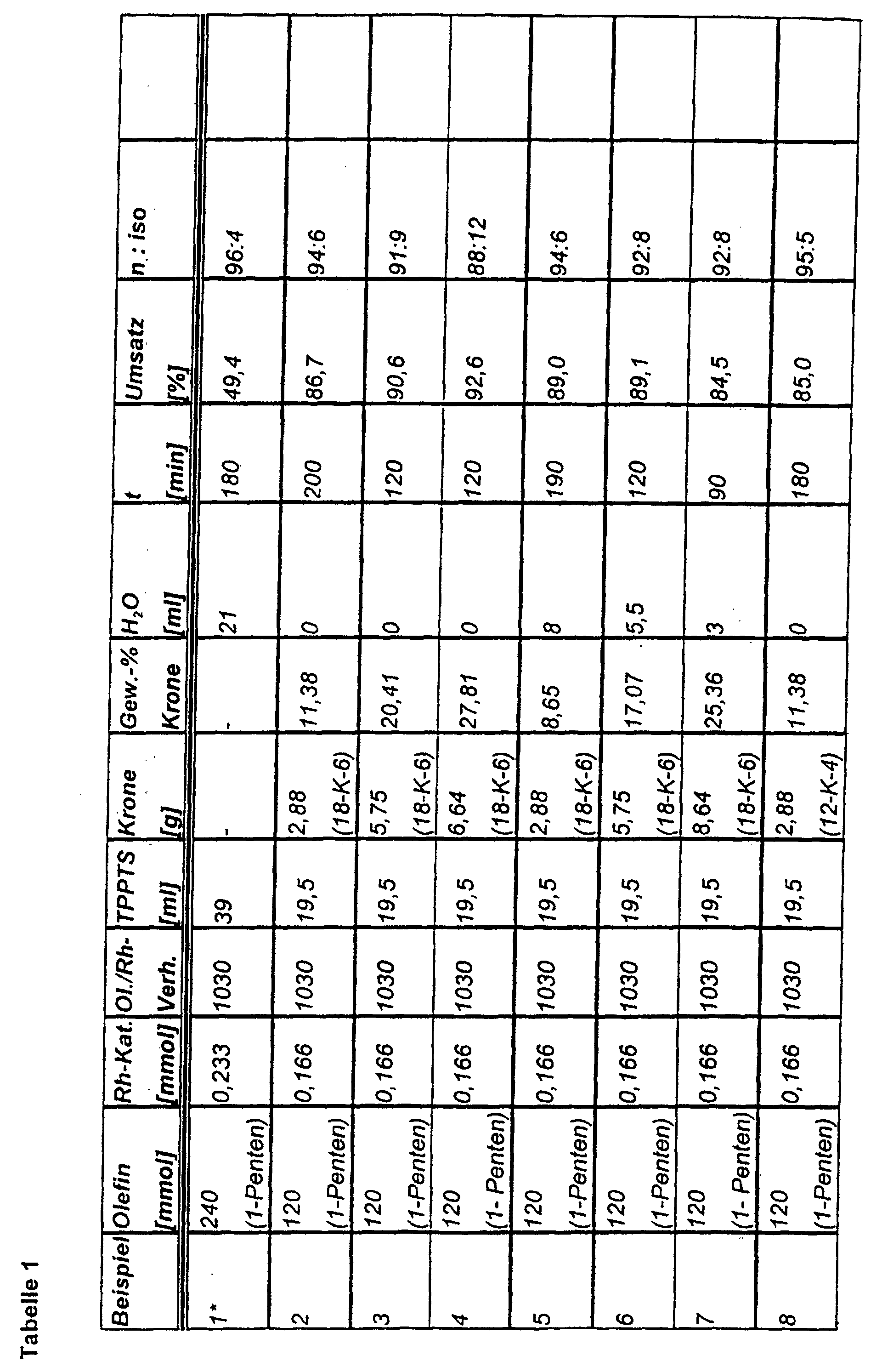

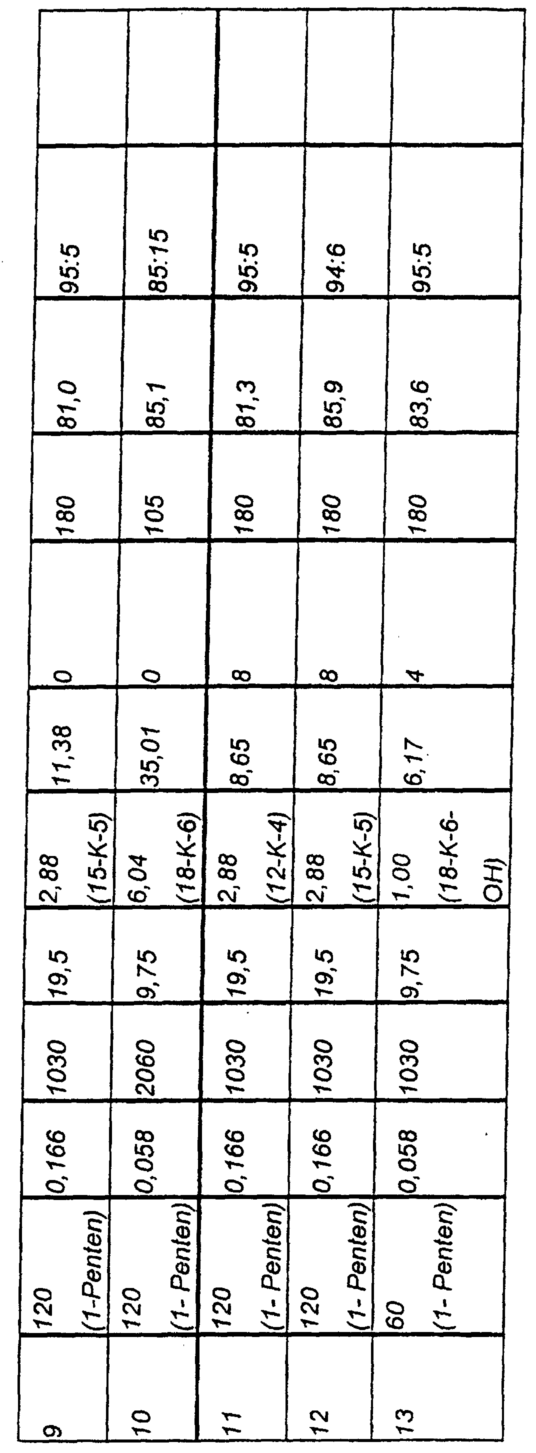

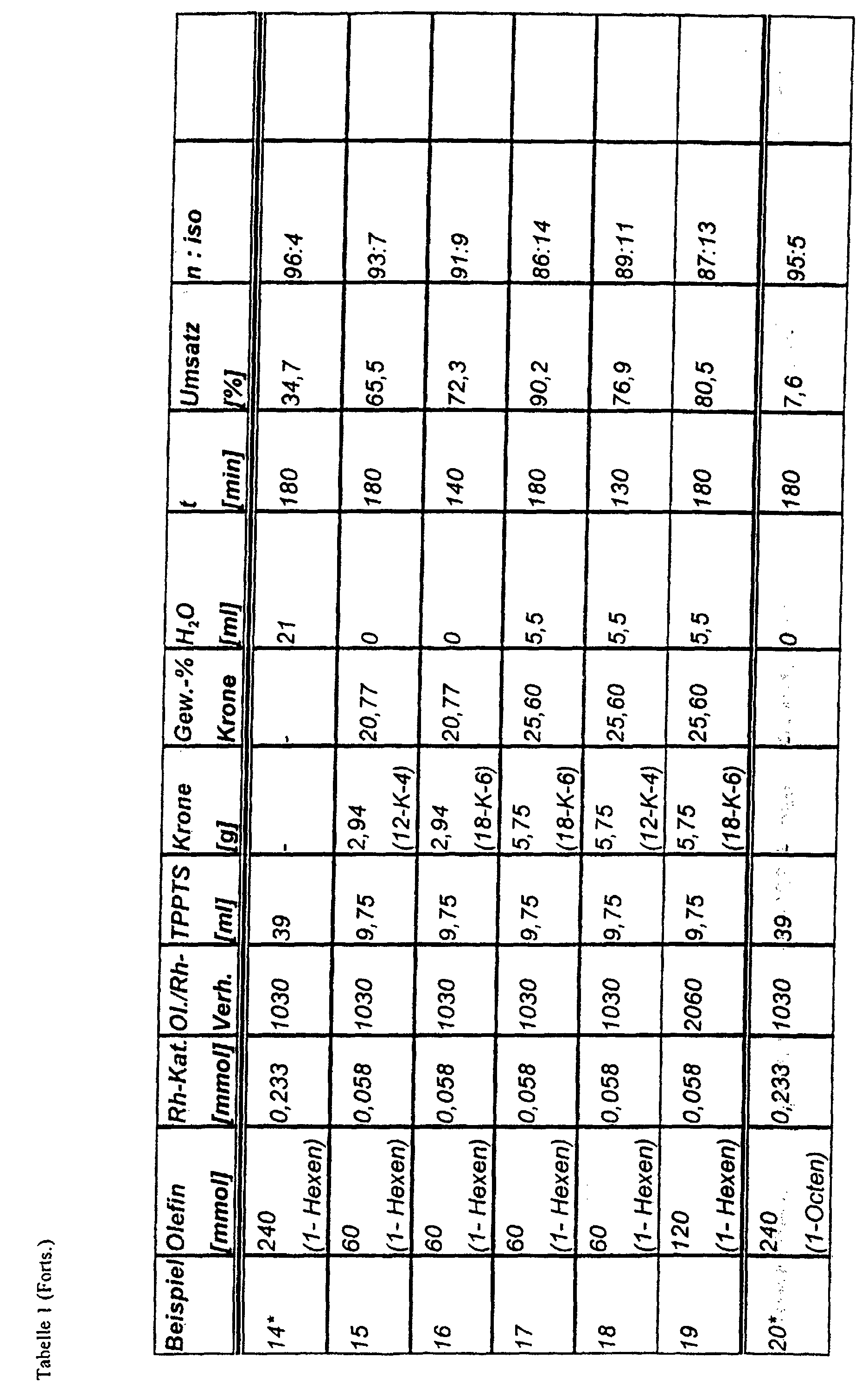

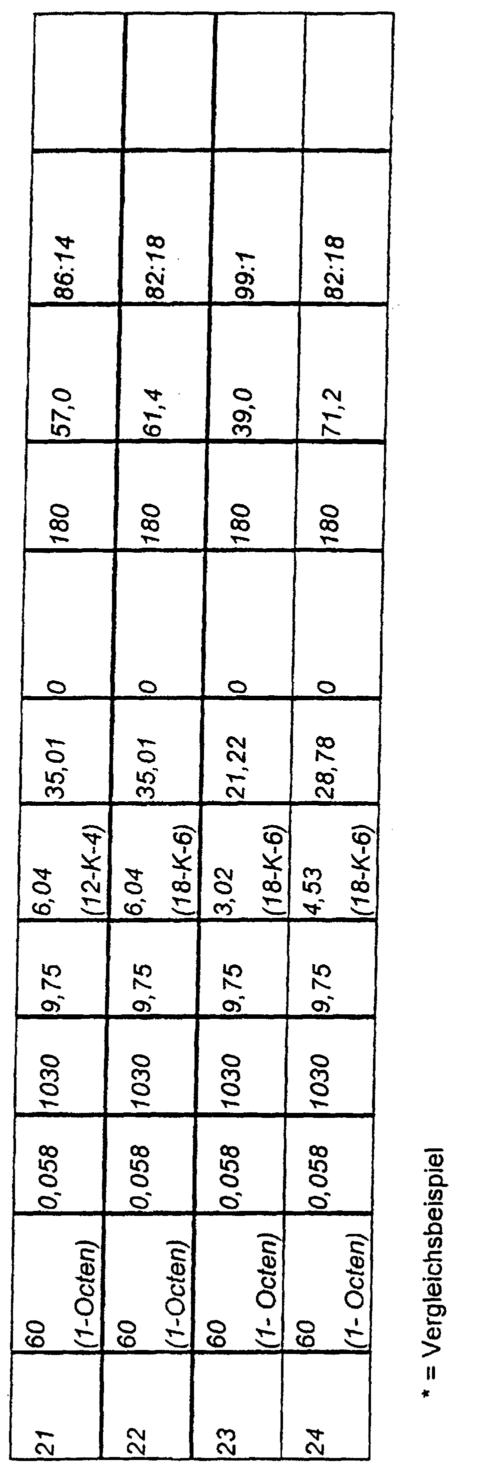

## Patentansprüche

1. Verfahren zur Herstellung von Aldehyden durch Umsetzung eines Olefins mit 3 bis 12 Kohlenstoffatomen mit Wasserstoff und Kohlenmonoxid in Gegenwart eines Rhodiumkatalysators, **dadurch gekennzeichnet, daß**
(a) der Rhodiumkatalysator in einer wäßrigen Phase vorliegt, wobei die wäßrige Phase
i) Rhodium in elementarer oder gebundener Form;
ii) ein trisulfoniertes Triarylphosphin der Formel (I) 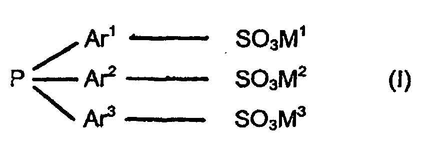 in der
• Ar¹, Ar² und Ar³ unabhängig voneinander jeweils einen Phenyl-, Naphthyl-, Biphenyl-, Phenylnaphthyl- oder Binaphthylrest darstellen;
• M¹, M² und M³ unabhängig voneinander jeweils ein Alkalimetallion oder ein Ammoniumion darstellen, und
iii) einen Kronenether enthält;
und
(b) das Olefin unter Reaktionsbedingungen in einer flüssigen organischen Phase vorliegt, die mit der wäßrigen Phase nicht mischbar ist.

2. Verfahren nach Anspruch 1, **dadurch gekennzeichnet, daß** das trisulfonierte Triarylphosphin der Formel (I) Trinatrium-tris(m-sulfophenyl)phosphin ist.

3. Verfahren zur Herstellung von Aldehyden durch Umsetzung eines Olefins mit 3 bis 12 Kohlenstoffatomen mit Wasserstoff und Kohlenmonoxid in Gegenwart eines Rhodiumkatalysators, **dadurch gekennzeichnet, daß**
(a) der Rhodiumkatalysator in einer wäßrigen Phase vorliegt, wobei die wäßrige Phase
i) Rhodium in elementarer oder gebundener Form;
ii) ein trisulfoniertes Triarylphosphin der Formel (II) 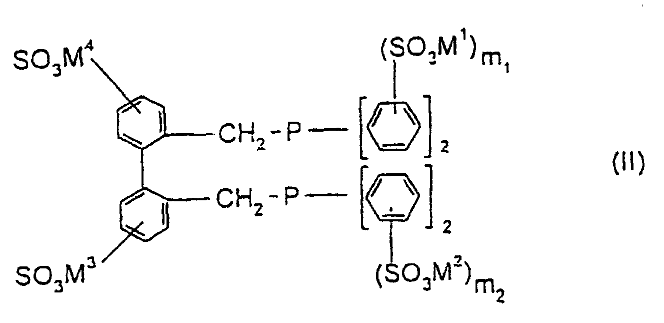 in der
• m¹ und m² den Wert 0 oder 1 haben können, wobei die Summe von m¹ und m² mindestens 1 beträgt; und
• M¹, M², M³ und M⁴ unabhängig voneinander jeweils ein Alkalimetallion oder ein Ammoniumion darstellen, und
iii) einen Kronenether enthält;
und
(b) das Olefin unter Reaktionsbedingungen in einer flüssigen organischen Phase vorliegt, die mit der wäßrigen Phase nicht mischbar ist.

4. Verfahren zur Herstellung von Aldehyden durch Umsetzung eines Olefins mit 3 bis 12 Kohlenstoffatomen mit Wasserstoff und Kohlenmonoxid in Gegenwart eines Rhodiumkatalysators, **dadurch gekennzeichnet, daß**
(a) der Rhodiumkatalysator in einer wäßrigen Phase vorliegt, wobei die wäßrige Phase
i) Rhodium in elementarer oder gebundener Form;
ii) ein trisulfoniertes Triarylphosphin der Formel (III) 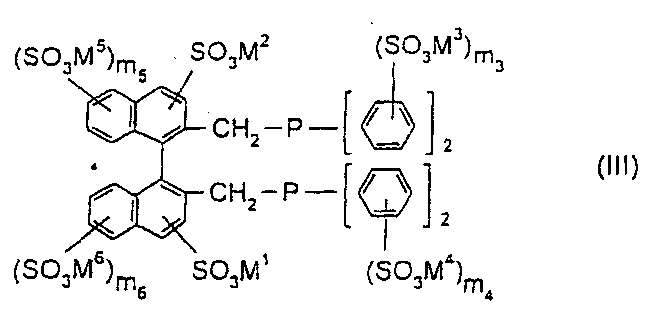 in der
• m₃, m₄, m₅ und m₆ den Wert 0 oder 1 haben können, wobei die Summe von m₃, m₄, m₅ und m₆ mindestens 2 beträgt; und
• M¹, M², M³, M⁴, M⁵ und M⁶ unabhängig voneinander jeweils ein Alkalimetallion oder ein Ammoniumion darstellen; und
iii) einen Kronenether enthält;
und
(b) das Olefin unter Reaktionsbedingungen in einer flüssigen organischen Phase vorliegt, die mit der wäßrigen Phase nicht mischbar ist.

5. Verfahren nach einem der vorherigen Ansprüche, **dadurch gekennzeichnet, daß** der Kronenether ausgewählt ist aus der Gruppe von 12-Krone-4, 15-Krone-5, 18-Krone-6 und Hydroxymethyl-18-Kone-6.

6. Verfahren nach einem der vorherigen Ansprüche, **dadurch gekennzeichnet, daß** das Olefin ausgewählt ist aus der Gruppe von aliphatischen, cycloaliphatischen und araliphatischen Olefinen, insbesondere aus aliphatischen α-Olefinen und cycloaliphatischen Olefinen.

7. Verfahren nach einem der vorherigen Ansprüche, **dadurch gekennzeichnet, daß** das Olefin ausgewählt ist aus der Gruppe von Propen, 1-Buten, 1-Penten, 1-Hexen, 1-Hepten, 1-Octen, 1-Nonen, 1-Decen, 1-Undecen und 1-Dodecen.

8. Verfahren nach einem der vorherigen Ansprüche, **dadurch gekennzeichnet, daß** das Olefin höchstens 10, vorzugsweise höchstens 8 Kohlenstoffatome aufweist.

9. Verfahren nach einem der vorherigen Ansprüche, **dadurch gekennzeichnet, daß** das Verhältnis von Rhodium zum Liganden zwischen 1:10 und 1:1000, vorzugsweise zwischen 1:50 und 1:200, liegt.

10. Verfahren nach einem der vorherigen Ansprüche, **dadurch gekennzeichnet, daß** die wäßrige Phase 20 bis 500 ppm, vorzugsweise 30 bis 150 ppm, insbesondere 40 bis 100 ppm, Rhodium enthält.

11. Verfahren nach einem der vorherigen Ansprüche, **dadurch gekennzeichnet, daß** die wäßrige Phase 2x10⁻⁶ bis 5x10⁻² Mol Rhodium pro Mol olefinischer Verbindung enthält.

12. Verfahren nach einem der vorherigen Ansprüche, **dadurch gekennzeichnet, daß** der Druck während der Reaktion zwischen 20 bis 150 bar, vorzugsweise zwischen 30 und 80 bar, liegt.

13. Verfahren nach einem der vorherigen Ansprüche, **dadurch gekennzeichnet, daß** die Temperatur während der Reaktion zwischen 20 und 170°C, vorzugsweise zwischen 100 und 140°C, liegt.

## Claims

1. A process for the production of aldehydes by the reaction of olefins of 3 to 12 carbon atoms with hydrogen and carbon monoxide in the presence of a rhodium catalyst, wherein
(a) the rhodium catalyst is present in an aqueous phase comprising
i) rhodium in elementary or bound form;
ii) a trisulfonated triarylphosphine of the formula (I) 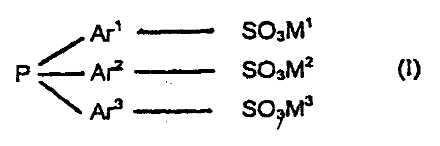 in which
• Ar¹, Ar² and Ar³ are individually selected from the group consisting of phenyl, naphthyl, biphenyl, phenylnaphthyl, binaphthyl;
• M¹, M² and M³ are individually alkali metal ion or ammonium ion; and
iii) a crown ether;
and
(b) the olefin under the reaction conditions is present in a liquid organic phase not miscible with the aqueous phase.

2. The process as claimed in claim 1, wherein the trisulfonated triarylphosphine of the formula I is trisodium tris(m-sulfophenyl)phosphine.

3. A process for the production of aldehydes by the reaction of olefins of 3 to 12 carbon atoms with hydrogen and carbon monoxide in the presence of a rhodium catalyst, wherein
(a) the rhodium catalyst is present in an aqueous phase comprising
i) rhodium in elementary or bound form;
ii) a trisulfonated triarylphosphine of the formula (II) 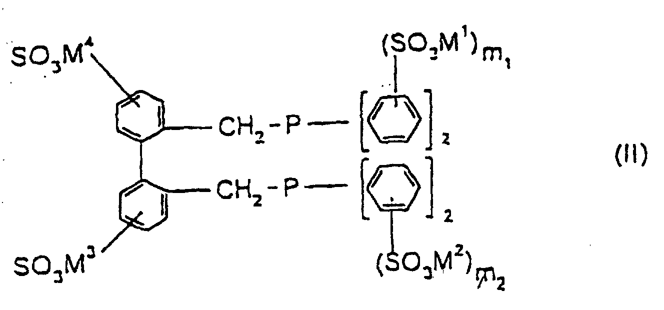 in which
• m¹ and m² are 0 or 1 and the sum of m¹ and m² is at least 1; and
• M¹, M², M³ and M⁴ are individually an alkali metal ion or ammonium ion; and
iii) a crown ether;
and
(b) the olefin under the reaction conditions is present in a liquid organic phase not miscible with the aqueous phase.

4. A process for the production of aldehydes by the reaction of olefins of 3 to 12 carbon atoms with hydrogen and carbon monoxide in the presence of a rhodium catalyst, wherein
(a) the rhodium catalyst is present in an aqueous phase comprising:
i) rhodium in elementary or bound form;
ii) a trisulfonated triarylphosphine of the formula (III) 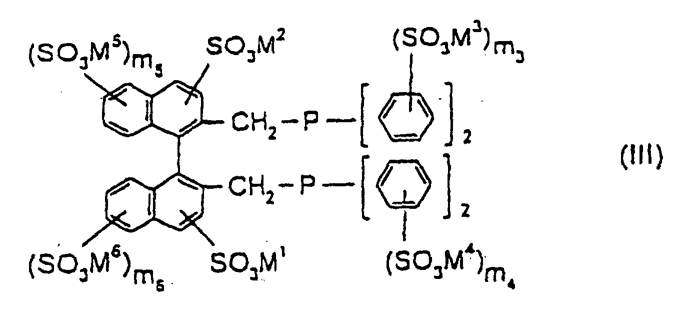 in which
• m₃, m₄, m₅ and m₆ are individually 0 or 1 and the sum of m₃, m₄, m₅ and m₆ is at least 2; and
• M¹, M², M³, M⁴, M⁵ and M⁶ are individually alkali metal ion or ammonium ion; and
iii) a crown ether;
and
(b) the olefin under the reaction conditions is present in a liquid organic phase not miscible with the aqueous phase.

5. The process as claimed in one of the preceding claims, wherein the crown ether is selected from the group consisting of 12-crown-4, 15-crown-5, 18-crown-6 and hydroxymethyl-18-crown-6.

6. The process as claimed in one of the preceding claims, wherein the olefin is selected from the group consisting of aliphatic, cycloaliphatic and araliphatic olefins, in particular of aliphatic α-olefins and cycloaliphatic olefins.

7. The process as claimed in one of the preceding claims, wherein the olefin is selected from the group consisting of propene, 1-butene, 1-pentene, 1-hexene, 1-heptene, 1-octene, 1-nonene, 1-decane, 1-undecene and 1-dodecene.

8. The process as claimed in one of the preceding claims, wherein the olefin has maximally 10 carbon atoms, preferably the olefin has at most 8 carbon atoms.

9. The process as claimed in one of the preceding claims, wherein the ratio of rhodium to ligand is between 1:10 and 1:1000, preferably between 1:50 and 1:200.

10. The process as claimed in one of the preceding claims, wherein the aqueous phase contains 20 to 500 ppm, preferably 30 to 150 ppm, in particular 40 to 100 ppm of rhodium.

11. The process as claimed in one of the preceding claims, wherein the aqueous phase contains 2x10⁻⁶ to 5x10⁻² mol of rhodium per mol of olefinic compound.

12. The process as claimed in one of the preceding claims, wherein the pressure during the reaction is between 20 and 150 bars, preferably between 30 and 80 bars.

13. The process as claimed in one of the preceding claims, wherein the temperature during the reaction is between 20 and 170°C, preferably between 100 and 140°C.

## Revendications

1. Procédé de préparation d'aldéhydes par réaction d'une oléfine ayant 3 à 12 atomes de carbone avec l'hydrogène et le monoxyde de carbone en présence d'un catalyseur au rhodium, **caractérisé en ce que**
(a) le catalyseur au rhodium est présent dans une phase aqueuse, la phase aqueuse comprenant:
i) le rhodium sous forme élémentaire ou liée ;
ii) une triarylphosphine trisulfonée de formule (I) 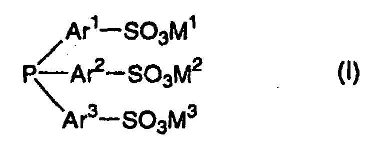 dans laquelle
• Ar¹, Ar² et Ar³ indépendamment l'un de l'autre représentent un groupe phényle, naphtyle, biphényle, phénylnaphtyle ou binaphtyle;
• M¹, M² et M³ indépendamment l'un de l'autre représentent chacun un ion de métal alcalin ou un ion ammonium ; et
iii) un éther couronne, et
(b) I'oléfine dans les conditions réactionnelles est présente dans une phase organique liquide qui n'est pas miscible avec la phase aqueuse.

2. Procédé selon la revendication 1, **caractérisé en ce que** la triarylphosphine trisulfonée de formule (I) est la tris(m-sulfophényl)phosphine trisodique.

3. Procédé de préparation d'aldéhydes par réaction d'une oléfine ayant 3 à 12 atomes de carbone avec l'hydrogène et le monoxyde de carbone en présence d'un catalyseur au rhodium, **caractérisé en ce que**
(a) le catalyseur au rhodium est présent dans une phase aqueuse, la phase aqueuse comprenant:
i) le rhodium sous forme élémentaire ou liée ;
ii) une triarylphosphine trisulfonée de formule (II) 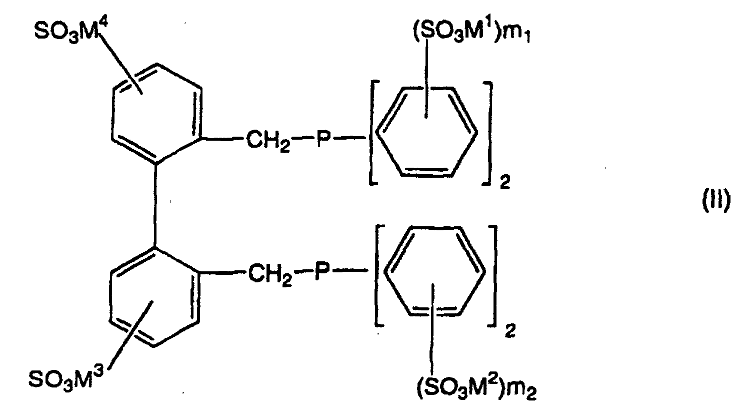 dans laquelle
• m¹ et m² peuvent avoir la valeur de 0 ou 1, la somme de m¹ et m² étant d'au moins 1, et
• M¹, M² M³ et M⁴ indépendamment l'un de l'autre représentent chacun un ion de métal alcalin ou un ion ammonium, et
iii) un éther couronne ; et
(b) l'oléfine dans les conditions réactionnelles est présente dans une phase organique liquide qui n'est pas miscible avec la phase aqueuse.

4. Procédé de préparation d'aldéhydes par réaction d'une oléfine ayant 3 à 12 atomes de carbone avec l'hydrogène et le monoxyde de carbone en présence d'un catalyseur au rhodium, **caractérisé en ce que**
(a) le catalyseur au rhodium est présent dans une phase aqueuse, la phase aqueuse comprenant :
i) le rhodium sous forme élémentaire ou liée ;
ii) une triarylphosphine trisulfonée de formule (III) 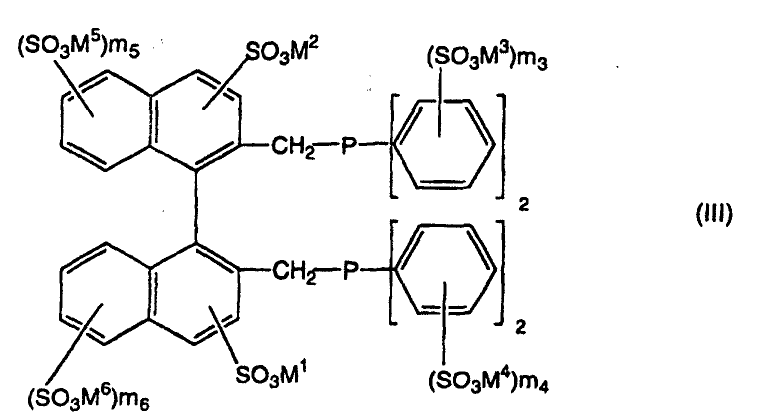 dans laquelle
• m₃, m₄, m₅ et m₆ peuvent avoir la valeur 0 ou 1, la somme de m₃, m₄, m₅ et m₆ étant d'au moins 2, et
• M¹, M² M³, M⁴, M⁵ et M⁶ indépendamment l'un de l'autre représentent chacun un ion de métal alcalin ou un ion ammonium, et
iii) un éther couronne ; et
(b) l'oléfine dans les conditions réactionnelles est présente dans une phase organique liquide qui n'est pas miscible avec la phase aqueuse.

5. Procédé selon l'une quelconque des revendications précédentes, **caractérisé en ce que** l'éther couronne est choisi dans le groupe formé par la 12-couronne -4, la 15-couronne-5, la 18-couronne-6 et l'hydroxyméthyl-18-couronne-6.

6. Procédé selon l'une quelconque des revendications précédentes, **caractérisé en ce que** l'oléfine est choisie dans le groupe des oléfines aliphatiques, cycloaliphatiques et araliphatiques, en particulier les α-oléfines aliphatiques et les oléfines cycloaliphatiques.

7. Procédé selon l'une quelconque des revendications précédentes, **caractérisé en ce que** l'oléfine est choisie dans le groupe formé par le propène, le 1-butène, le 1-pentène, le 1-hexène, le 1-heptène, le 1-octène. le 1-nonène, le 1-décène, le 1-undécène et le 1-dodécène.

8. Procédé selon l'une quelconque des revendications précédentes, **caractérisé en ce que** l'oléfine présente au maximum 10, de préférence au maximum 8 atomes de carbone.

9. Procédé selon l'une quelconque des revendications précédentes, **caractérisé en ce que** le rapport du rhodium au ligand est compris entre 1:10 et 1:1000, de préférence entre 1 : 50 et 1 : 200.

10. Procédé selon l'une quelconque des revendications précédentes, **caractérisé en ce que** la phase aqueuse contient 20 à 500 ppm, de préférence 30 à 150 ppm, en particulier 40 à 100 ppm, de rhodium.

11. Procédé selon l'une quelconque des revendications précédentes, **caractérisé en ce que** la phase aqueuse contient 2x10⁻⁶ à 5x10⁻² moles de rhodium par mole de composé oléfinique.

12. Procédé selon l'une quelconque des revendications précédentes, **caractérisé en ce que** la pression pendant la réaction est comprise entre 20 et 150 bars, de préférence 30 et 80 bars.

13. Procédé selon l'une quelconque des revendications précédentes, **caractérisé en ce que** la température pendant la réaction est comprise entre 20 et 170°C, de préférence entre 100 et 140°C.
